# EUROPEAN PATENT SPECIFICATION

(11) **EP 2 385 034 B1**
(45) Date of publication and mention of the grant of the patent: **02.12.2015**
(21) Application number: 11164806.9
(22) Date of filing: 04.05.2011
(51) Int. Cl.: C07C 231/12, C07C 233/13, C07C 233/31, C07C 233/75, C07C 271/48

(54) **Process for the preparation of 1-nitro-4-oxobutanylamides**
Verfahren zur Synthese von 1-Nitro-4-oxobutanylamiden
Procédé de préparation de 1-nitro-4-oxobutanylamides

(30) Priority: 05.05.2010 SK 50132010
(43) Date of publication of application: 09.11.2011
(73) Proprietor: Synkola, s.r.o., 842 15 Bratislava (SK)
(72) Inventor: Rehak, Juraj, Ing., PhD., 027 41, Oravsky Podzamok (SK); Lu, Gui, Prof., Guangzhou University City, Guangzhou 510006 (CN); Durmis, Julius, Ing., PhD., 841 02, Bratislava (SK); Weng, Jiang, Guangzhou University City, Guangzhou 510006 (CN); Brath, Henrich, Ing., 951 02, Hostova 67 (SK); Li, Yong-Bo, Guanghzou 510006 (CN); Hutka, Martin, RNDr., PhD., 811 07, Bratislava (SK); Toma, Stefan, RNDr., Prof., 841 05, Bratislava (SK); Latika, Attila, 831 03 Bratislava (SK)
(74) Representative: Bachrata, Magdaléna

(56) References cited:
- CN-A- 101 735 140
- SHAOLIN ZHU ET AL: "Organocatalytic Michael Addition of Aldehydes to Protected 2-Amino-1-Nitroethenes: The Practical Syntheses of Oseltamivir (Tamiflu) and Substituted 3-Aminopyrrolidines", ANGEWANDTE CHEMIE INTERNATIONAL EDITION, vol. 49, no. 27, 17 May 2010 (2010-05-17), pages 4656-4660, XP55028907, ISSN: 1433-7851, DOI: 10.1002/anie.201001644
- XIONG-LI LIU ET AL: "Amino-Indanol-Catalyzed Asymmetric Michael Additions of Oxindoles to Protected 2-Amino-1-nitroethenes for the Synthesis of 3,3'-Disubstituted Oxindoles Bearing [alpha],[beta]-Diamino Functionality", THE JOURNAL OF ORGANIC CHEMISTRY, vol. 76, no. 10, 4 April 2011 (2011-04-04) , pages 4008-4017, XP55028911, ISSN: 0022-3263, DOI: 10.1021/jo2004378
- ZOLTÁN MUCSI ET AL: "A Quantitative Scale for the Extent of Conjugation of the Amide Bond. Amidity Percentage as a Chemical Driving Force", THE JOURNAL OF PHYSICAL CHEMISTRY A, vol. 111, no. 50, 1 December 2007 (2007-12-01), pages 13245-13254, XP55028983, ISSN: 1089-5639, DOI: 10.1021/jp0759325
- JIANG WENG ET AL: "A Practical and Azide-Free Synthetic Approach to Oseltamivir from Diethyl d-Tartrate", THE JOURNAL OF ORGANIC CHEMISTRY, vol. 75, no. 9, 15 April 2010 (2010-04-15) , pages 3125-3128, XP55029163, ISSN: 0022-3263, DOI: 10.1021/jo100187m

## Description

### Field of the Invention

The invention relates to the process for the preparation of 1-nitro-4-oxobutanylamides and opposite enantiomers thereof suitable for the preparation of cyclohexene derivatives, which are used as intermediates for the preparation of antivirals. The invention further relates to 2-nitroethenylamide compounds and the process for the preparation thereof.

### Background of the Invention

In literature only 1-nitro-3-pentyloxy-4-oxobutanylacetamide and the process for the preparation thereof is known, which process consists of 8 steps, as described in JOC 75, (20), 3125 (2010*)*, and is based on enantiomerically pure diethylester of tartaric acid according to the following scheme:

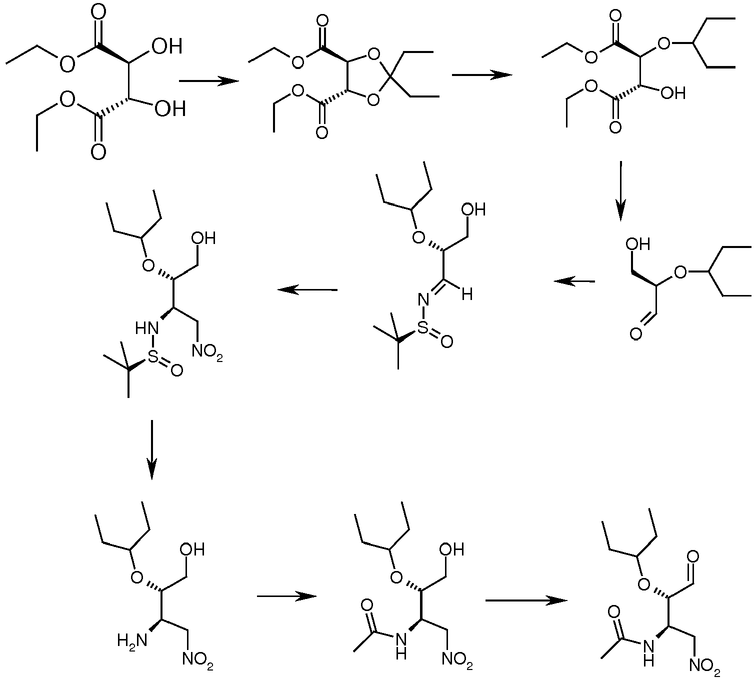

The above described process is disadvantageous due to a long 8-step path with low total yields, and the starting material is a rather expensive enantiomerically pure compound.

The aim of this invention are also nitroethenylacylamides and process for their preparation. In literature is only one reference to these nitroacetylamides. in J. Phys. Chem. A 2007, 111, 13245-13254. Authors disclose a correlation between the computed amidity percentage and their resonance energy values, proton afinities and reactivity in nucleofilic addition reaction by computer method. Computation were carried out using the Gausian03 program package. The studied amides were only drawn and 2-nitroetrhenylacetamide given in this publication with structure 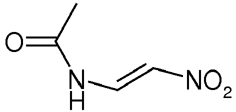 was never prepared and not described nor in this publication nor in other documents before and it is not known the method of preparation of such compound and not known application of such amide. Beside this above drawn compound can exist in two form Z- and E-form.

The aim of this invention is to prepare 1-nitro-4-oxobutanylamides and opposite enantiomers thereof by a process removing deficiencies of the above known multistep process.

### Summary of the Invention

Unless otherwise specified, the below cited terms used in description of the application have the following meaning: "Alkyl" means a linear saturated hydrocarbon radical or branched saturated hydrocarbon radical. Such alkyl groups include methyl, ethyl, n-propyl, 2-propyl, n-butyl, 2-methylpropyl, tert-butyl, as well as isomer pentyl, hexyl, octyl, nonyl, decyl, undecyl and dodecyl.
"Cycloalkyl" means a cyclic hydrocarbon radical with at least three carbon atoms, preferably 3 to 6 carbon atoms.

The above deficiencies can be removed to a considerable extent by preparing 1-nitro-4-oxobutanylamides and opposite enantiomers thereof under the process according to the present invention.

The invention provides the process for the preparation of 1-nitro-4-oxobutanylamides of general formula Ia and Ib 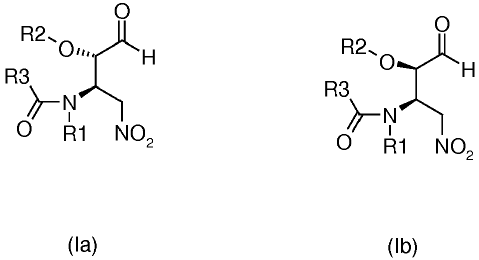 and opposite enantiomers thereof,
wherein R1 is H, allyl, alkyloxycarbonyl, arylalkyloxycarbonyl, alkoxyphenylalkyl with a number of carbon atoms up to 24, or the same as in R2, R2 represents alkyl, cycloalkyl or phenylalkyl with up to 24 carbon atoms, R3 represents alkyl, alkylphenyl, phenylalkyl, phenyl, substituted phenyl or alkoxyphenylalkyl with up to 24 carbon atoms, or wherein R3 is methyl when R1 is 4-methoxyphenylmethyl,
by reaction of aldehyde of general formula II 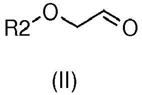 wherein R2 is as cited above,
with 2-nitroethenylamide of general formula IIIa and/or IIIb 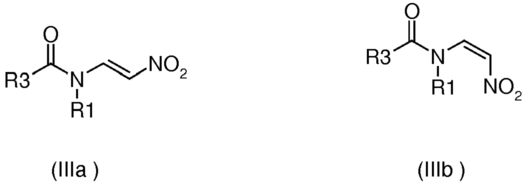 wherein R1 and R3 are defined as above,
in the presence of organic catalysts and co-catalysts in organic solvent, water, in solution of inorganic salts in water or in the mixture of organic solvents and water or organic solvents and solutions of inorganic salts in water at the temperature between -20 and +50 °C.

It was found out that R1 is preferably H, R2 is preferably 1-ethylpropyl and R3 is preferably methyl.

An organic catalyst is preferably a pyrrolidine derivative of general formula IV and V, 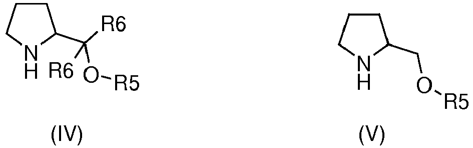 wherein R5 is alkyl with 1 to 4 carbon atoms or trimethylsilyl and R6 is phenyl or substituted phenyl, preferably (2S)-2-(diphenyl[(trimethylsilyl)oxy]methyl)pyrrolidine or (2*R*)-2-(diphenyl[(tri-methylsilyl)oxy]methyl)pyrrolidine.

Carboxylic acids with 1 to 12 carbon atoms or carboxylic acids substituted with halogen, aromatic acids, phenylalkane carboxylic acids, cycloalkanecarboxylic acids with 6 to 12 carbon atoms or several other organic acids with 1 to 12 carbon atoms, preferably monochloroacetic acid, bromoacetic acid or iodoacetic acid, can be used as co-catalysts.

Preferred solvents comprise alcohols with 1 to 5 carbon atoms, water, or mixture of water with dichloromethane or chloroform or mixture of solution of inorganic salts, such as NaCl in water with dichloromethane or chloroform, temperature is preferably between -15 and 25 °C.

Examples of compounds of general formula Ia and Ib are, inter alia:
2-methyl-N-[(2R, 3S)-1-nitro-4-oxo-3-(pentane-3-yloxy)butane-2-yl]propanamide and enantiomer thereof,
2-methyl-N-[(2S, 3R)-1-nitro-4-oxo-3-(pentane-3-yloxy)butane-2-yl]propanamide and its enantiomer,
2-methyl-N-[(2R, 3R)-1-nitro-4-oxo-3-(pentane-3-yloxy)butane-2-yl]propanamide and enantiomer thereof,
2-methyl-N-[(2S, 3S)-1-nitro-4-oxo-3-(pentane-3-yloxy)butane-2-yl]propanamide and enantiomer thereof,
2,2-dimethyl-N-[(2R, 3S)-1-nitro-4-oxo-3-(pentane-3-yloxy)butane-2-yl]propanamide and enantiomer thereof,
N-[(2R,3S)-1-nitro-4-oxo-3-(pentane-3-yloxy)butane-2-yl]acetamid and enantiomer thereof.

The invention further provides 2-nitroethenylamides of general formula IIIa and Illb 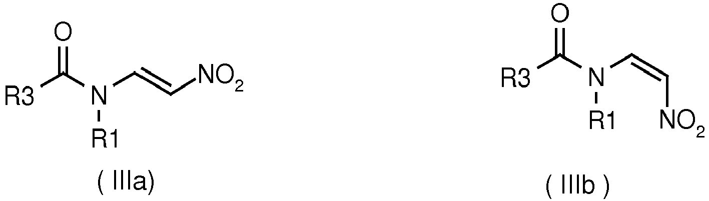 wherein R1 is H, allyl, alkyloxycarbonyl, arylalkyloxycarbonyl with a number of carbon atoms up to 24 or alkyl, cycloalkyl or phenylalkyl with up to 24 carbon atoms, and R3 represents alkyl, alkylphenyl, phenylalkyl or phenyl, alkoxyphenylalkyl with up to 24 carbon atoms, or wherein R3 is methyl when R1 is 4-methoxyphenylmethyl.
It was found out that R1 is preferably H and R3 is preferably methyl.

Examples of compounds of general formula IIIa and IIIb are, inter alia:
N-[(Z)-2-nitroethenyl]acetamide, N-[(E)-2-nitroethenyl]acetamide, 2-methyl-N-[(E)-2-nitro-ethenyl]propanamide, 2,2-dimethyl-N-[(Z)-2-nitroethenyl]propanamide, N-[(4-metoxyphenyl)methyl]-N-[(E)-2-nitroethenyl]acetamide.

The invention further provides the process for the preparation of 2-nitroethenylamides of general formula IIIa and IIIb or mixtures thereof 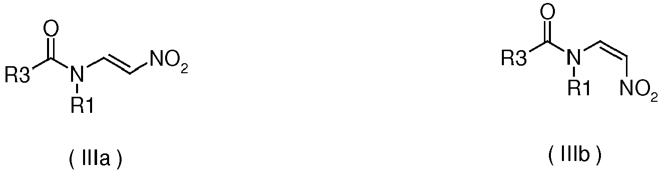 wherein R1 and R3 are as cited above,
by reaction of 2-nitroethenylamines of general formula VIa and/or VIb, 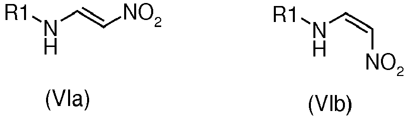 wherein R1 is defined as above,
with carboxylic acids or detivatives thereof with up to 24 carbon atoms. Carboxylic acid derivative is preferably acetic acid anhydride.

1-nitro-4-oxobutanylamides of general formula Ia and Ib are used for the preparation of cyclohexene derivatives of general formula A, B, C, D 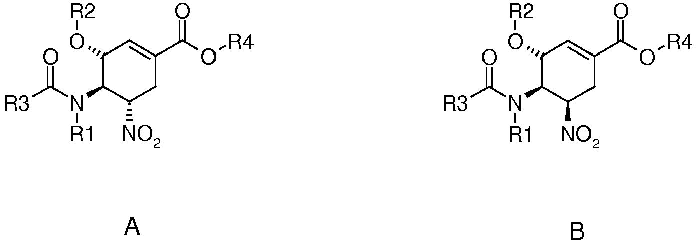 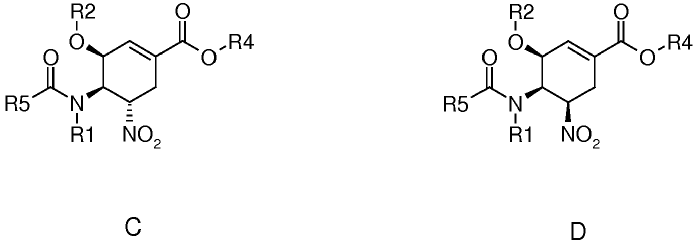 wherein R1, R2 and R3 are as cited above, and R4 represents alkyl with up to 24 carbon atoms, which can be prepared in one step by a cascade reaction, and by the subsequent reduction of nitrogroup (for example by the procedure described in JOC 75, (20), 3125 (2010*)* it is possible to prepare potential inhibitors of neuraminidase of general formula E 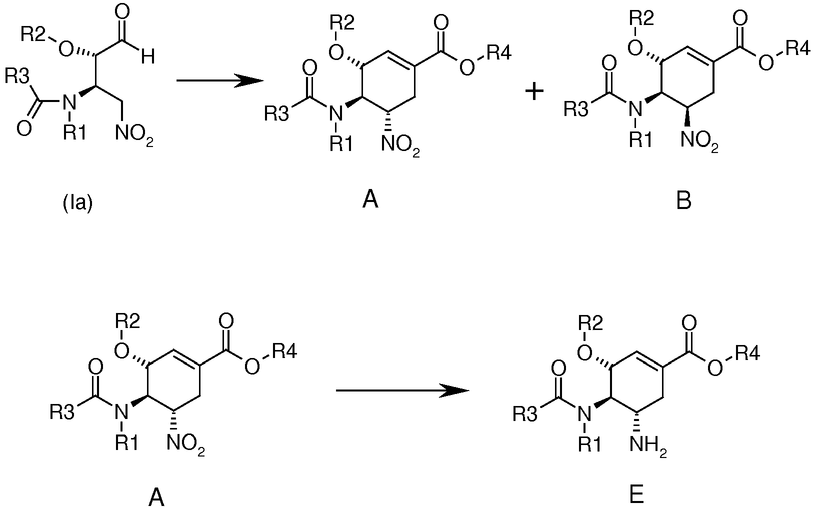 wherein R1, R2, R3 and R4 are as cited above,
and salts thereof.

Cyclohexene derivatives of general formula E, particularly (3R,4R,5S)-4-acetamide-5-amino-3-(1-ethylpropoxy)-1-cyclohexene-1-carboxylic acid ethylester and salts thereof, are inhibitors of an enzyme known as neuraminidase (J.C. Rohloff at al., J. Org. Chem., 1998, 63, 4545-4550*,* WO 98/07685*).*

There are several methods for the preparation of effective inhibitors of neuraminidase based on cyclohexenecarboxylic acid esters, such as *(3R, 4R, 5S)-4-acetamide-5-amino-3-(1-ethylpropoxy)-1-cyclohexene-1-carboxylic acid ethylester.* However, all these methods are multistep synthesises, sometimes also with relatively dangerous chemical substances, such as azides, or with substances having unpleasant smell for living and working environment, such as thiols *(*Hayato Ishikawa, Takaki Suzuki, and Yujiro Hayashi Angew. Chem. Int. Ed. 2009, 48, 1304-1307 *and literature cited in this document).* Moreover, relatively expensive chemicals, such (-)-Shikimic acid or (-)-Quinic acid, are used in most of the above methods.

Aldehydes of general formula II can easily be prepared by known methods, for example according to Hayato Ishikawa, Takaki Suzuki, and Yujiro Hayashi Angew. Chem. Int. Ed. 2009, 48, 1304-1307*.*

Process for the preparation of the compound of general formula VIa and/or Vlb is described for example in Krowcynski A., Kozerski L., Synthesis 1983, 6, 489-491.

Process for the preparation of 1-nitro-4-oxobutanylamides of general formula Ia and Ib is advantageous in that it consists of one step, and thus it is simple and quick, brings high yields and high enantiomer excesses of the desired diastereoisomer.

### Examples

The following examples illustrate the present invention in more details, without being limitative.

*Note*: The numbered compounds with "r" represent racemic mixtures.

### Preparation of 1-nitro-4-oxobutanylamides

### Example 1

### Preparation of N-[(2S,3R and 2R,3S)-1-nitro-4-oxo-3-(pentane-3-yloxy)butane-2-yl]acetamide (1 r) and N-[(2S,3S and 2R, 3R)-1-nitro-4-oxo-3-(pentane-3-yloxy)butane-2-yl]acetamide (2r)

Monochloroacetic acid (0.2 equiv., 56 mg) and (*2R*)-2-(diphenyl[(trimethylsilyl)oxy]methyl)pyrrolidine (0.05 equiv., 48 mg) in methanol (5 ml) were added to the suspension of N - [(Z)-2-nitro-ethenyl]acetamide (3 mmol, 390 mg) and 1-ethylpropyloxyacetaldehyde (2 equiv., 780 mg) in methanol (10 ml) at room temperature. The mixture was stirred at room temperature for 16 hours. After the reaction was terminated, methanol was evaporated in the rotary vacuum evaporator and the crude product was separated by chromatography (silica gel, ethylacetate-hexane=1:1). A light brown oil (778 mg, 99%) as a mixture of diastereoisomers **1r** and **2r**, dr: *syn: anti* 45:55, ee: *syn*: 12%, *anti*: 9%) was obtained.
syn-aldehyde (**1r**): ¹H NMR, (300 MHz, CDCl₃) δ 9,65 (t, *J* = 0,6 Hz, 1H), 6,04 (bd, *J* = 8,5 Hz, 1 H), 5,11-5,02 (m, 1 H), 4,58 (d, *J* = 6,5 Hz, 2H), 4,08 (dd, *J* = 0,3, 3,3 Hz, 1 H), 3,41 (quint., *J* = 5,8 Hz, 1 H), 1,99 (s, 3H), 1,62-1,49 (m, 4H), 0,98-0,87 (m, 6H) MS *m*/*z* [M+H⁺] calculated for [C₁₁H₂₁N₂O₅]⁻: 261,30; found: 261,13.
anti-aldehyde (**2r**)**:** ¹H NMR, (300 MHz, CDCl₃) δ 9,61 (d, *J*= 3,1 Hz, 1 H), 6,11 (bd, *J* = 8,8 Hz, 1 H), 4,85-4,50 (m, 3H) 3,95 (dd, *J* = 3,1, 8,0 Hz, 1 H), 3,27 (quint., *J* = 5,5 Hz, 1 H), 2,00 (s, 3H), 1,57-1,42 (m, 4H), 0,95-0,84 (m, 6H). MS *m*/*z* [M+H⁺] calculated for [C₁₁H₂₁N₂O₅]⁻: 261,30; found: 261,13

### Example 2

### Preparation of 2-methyl-N-[(2S,3R and 2R,3S)-1-nitro-4-oxo-3-(pentane-3-yloxy)butane-2-yl]propanamide (3r) and 2-methyl-N-[(2S,3S and 2R,3R)-1-nitro-4-oxo-3-(pentane-3-yloxy)butane-2-yl]propane-amid (4r)

Monochloroacetic acid (0.4 equiv., 19 mg) and (2R)-2-(diphenyl[(trimethylsilyl)oxy]-methyl)pyrrolidine (0.1 equiv., 16 mg) in CHCl₃ (2 ml) were added to the suspension of 2-methyl-N-[(E)-2-nitroethenyl]propanamide (0.5 mmol, 79 mg) and 1-ethylpropyloxyacetaldehyde (1.5 equiv., 130 mg) in CHCl₃ (1 ml) and water (2 ml) at room temperature. The mixture was stirred at this temperature for 7 hours. After the reaction terminated, the saturated solution of NH₄Cl (3 ml) was added and an organic chloroform layer was separated. The residue was extracted 5x by chloroform (5x1 ml). The joined organic layers were dried over Na₂SO₄ and after filtration the solvent evaporated in the rotary vacuum evaporator. The crude product was separated by chromatography (silica gel, ethylacetate-hexane=1:1). A light brown oil **3r** and **4r** (140 mg, 97%: dr: *syn: anti* 52:48) was obtained.
*syn*-aldehyd (**3r**): 1 H NMR, (300 MHz, CDCl₃) δ 9,64 (t, J = 0,5 Hz, 1 H), 6.06 (bd, J = 8,5 Hz, 1 H), 11/05-01/05 (m, 1 h), 4.60 (d, J = 1,1 Hz, 1 H), 4.57 (d, J = 1,7 Hz, 1 H), 4.10 (dd, J = 0,3, 3,1 Hz, 1 H), 3.41 (quint., J = 5,7 Hz, 1 H), 2.37 (sept, J = 6,7 Hz, 1 H), 1.64-1.45 (m, 4 h), 1,16-1,11 (m, 6H), 0.98-0.87 (m, 6H) *MS: m*/*z* [M+H⁺] calculated for: [C₁₃H₂₅N₂O₅] = 289,1758; found: 289,16
anti-aldehyd (**4r**): 1 H NMR, (300 MHz, CDCl₃) δ 9,60 (d, J = 3,1 Hz, 1 H), 6.09 (bd, J = 8,7 Hz, 1 H), 4.82 (dd, J = 5,3, 13,3 Hz, 1 H), 4.76-4.68 (m, 1 H), 4.56 (dd, J = 3,6, 13,3 Hz, 1 H), 3.94 (dd, J = 3,1, 8,2 Hz, 1 H), 3.26 (quint., J = 5,7 Hz , 1 H), 2,38-2.36 (sept, J = 6,9 Hz, 1H), 1.61-1,46 (m, 4 h), 1,16-1,11 (m, 6H), 0.94-0,84 (m, 6H) *MS: m*/*z* [M+H⁺] calculated for: [C13H25N2O5] = 289,1758; found: 289,16

### Example 3

### Preparation of 2-methyl-N-[(2S,3R and 2R,3S)-1-nitro-4-oxo-3-(pentane-3-yloxy)butane-2-yl]propanamide (3r) and 2-methyl-N-[(2S,3S and 2R, 3R)-1-nitro-4-oxo-3-(pentane-3-yloxy)butane-2-yl]propane-amide (4r) in the presence of bromoacetic acid

The order of steps was the same as in Example 2: bromoacetic acid (0.2 equiv., 36.13 mg) dissolved in H₂O (5.2 ml) and (2*S*)-2-(diphenyl[(trimethylsilyl)-oxy]methyl)-pyrrolidine (0.1 equiv., 42.32 mg) in CDCl₃ (0.5 ml) were added to the suspension of 2-methyl-N-[(E)-2-nitroethenyl]propanamide (1.3 mmol, 205.61 mg) and pent-3-yloxyacetaldehyde (1.5 equiv., 253.87 mg) in CHCl₃ (4.7 ml) at 0 °C. The mixture was stirred for 8 hours at the temperature of 0 °C. After the reaction terminated, the saturated solution of NH₄Cl (28.5 ml) was added to the reaction mixture at the temperature of 0 °C. The mixture was further stirred at this temperature for 15 minutes. The reaction mixture was extracted 5x by chloroform, organic layers were dried over Na₂SO₄ and filtered. The crude product was evaporated in the rotary vacuum evaporator.

The products **3r** and **4r** (85.7 %) dr: *syn: anti*= 1.6:1, ee: *syn* 70.8 %, ee *anti* 21.8 % were isolated from the crude product (dark green oil, 415 mg, dr: *syn:anti* 2.1:1) by chromatography (silica gel, ethylacetate-hexane=2:1)

### Example 4

### Preparation of 2,2-dimethyl-N-[(2S,3R and 2R,3S)-1-nitro-4-oxo-3-(pentane-3-yloxy)butane-2-yl]propanamide (5r) and 2,2-dimethyl-N-[(2S,3S and 2R,3R)-1-nitro-4-oxo-3-(pentane-3-yloxy)butane-2-yl]propane-amide (6r)

Monochloracetic acid (0.4 equiv., 19 mg) and (2R)-2-(diphenyl[(trimethylsilyl)-oxy]methyl)pyrrolidine (0.1 equiv., 16 mg) in 2-propanol (2 ml) were added to the suspension of 2,2-dimethyl-N-(Z)-2-nitroethenyl]propanamide (0.5 mmol, 86 mg) and 1-ethylpropyloxyacetaldehyde (2 equiv., 130 mg) in 2-propanol (3 ml) at room temperature. The mixture was stirred at room temperature for 22 hours. After the reaction terminated, 2-propanol evaporated in the rotary vacuum evaporator and the crude product was separated by chromatography (silica gel, ethylacetate:hexane=1:2). A slighly brown oil (150 mg, 99%) of two diastereoisomers **5r** and **6r** dr: *syn:anti* 60:40 was obtained.
syn-aldehyd (**5r**): 1 H NMR, (300 MHz, CDCl₃) δ 9,63 (t, J = 0,6 Hz, 1 H), 6,28 (bd, J = 8,6 Hz, 1 H), 5,05-4,97 (m, 1 H), 4,60 (d, J = 1,4 Hz, 1 H), 4,57 (d, J = 1,4 Hz, 1 H), 4,11 (dd, J = 0,3, 3,5 Hz, 1 H), 3,42 (quint., J = 5,8 Hz, 1 H), 1,65-1,47 (m, 4H), 1,18 (s, 9H), 0,99 to 0,89 (m, 6H). *MS m*/*z* [M+H⁺] calculated for [C₁₄H₂₆N₂O₅]: 303,1914; found: 303,1811.
*anti*-aldehyd (**6r**): 1 H NMR, (300 MHz, CDCl₃) δ 9,60 (d, J = 3,0 Hz, 1 H), 6,32 (bd, J = 8,8 Hz, 1 H), 4,81 (dd, J = 5,2, 13,1 Hz, 1 H), 4,75-4,62 (m, 1 H), 4,56 (dd, J = 3,6, 13,1 Hz, 1 H), 3,94 (dd, J = 3,0, 8,0 Hz, 1 H), 3,26 (quint., J = 5,5 Hz, 1 H), 1,55-1,41 (m, 4H), 1,17 (s, 9H), 0,94-0,83 (m, 6H).MS *m*/*z* [M+H⁺] calculated for [C₁₄H₂₆N₂O₅]: 303,1914; found: 303,1811.

### Example 5

### Preparation of N-[(2S,3R)-1-nitro-4-oxo-3-(pentane-3-yloxy)butane-2-yl]acetamide (1 a) and N-[(2S,3S and 2R, 3R)-1-nitro-4-oxo-3-(pentane-3-yloxy)butane-2-yl]acetamide (2r)

Monochloracetic acid (0.4 equiv., 75 mg) and (2R)-2-(diphenyl[(trimethylsilyl)oxy]methyl)pyrrolidine (0.1 equiv., 65 mg) in CHCl₃ (4 ml) were added to the suspension of N-[(Z)-2-nitroethenyl]-acetamide (2 mmol, 260mg) and 1-ethylpropyloxyacetaldehyde (2) (1.5 equiv., 780 mg) in CHCl₃ (8 ml) and saturated solution of NaCl (8 ml) at room temperature. The mixture was stirred at this temperature for 16 hours. After the reaction terminated, the saturated solution of NH₄Cl (10 ml) was added and chloroform layer was separated. The residuum was extracted by chloroform (5x4ml) and joined organic layers were dried over Na₂SO₄ and filtrated. The filtrate evaporated in rotary vacuum evaporator and the residuum was separated by chromatography (silica gel, ethylacetate:hexane=1:1). A light brown oil of the enantiomer **1a** and diastereoisomer **2r** (390 mg, 75 %, dr: *syn*: *anti* 76:23, ee: *syn*: 92 %, *anti:* 38 %) was obtained.

### Example 6

### Preparation of N-[(2S,3R)-1-nitro-4-oxo-3-(pentane-3-yloxy)butane-2-yl]acetamide (1 a) and N-[(2S,3S and 2R, 3R)-1-nitro-4-oxo-3-(pentane-3-yloxy)butane-2-yl]acetamide (2r)

The order of steps was the same as in Example 5.

Monochloroacetic acid (0.2 equiv., 75 mg) and (2R)-2-(diphenyl[(trimethylsilyl)-oxy]methyl)pyrrolidine (0.1 equiv., 130 mg) in CHCl₃ (8 ml) were added to the suspension of N-[(*Z*)-2-nitroethenyl]-acetamide (4 mmol, 520mg) and 1-ethylpropyloxyacetaldehyde (1.5 equiv., 780 mg) in CHCl₃ (16 ml) and water (16 ml) at 0 °C. The mixture was stirred at this temperature for 5 hours. After the reaction terminated, the saturated solution of NH₄Cl (20 ml) was added and chloroform layer was separated. The residuum was extracted by chloroform (5x10ml) and joined organic layers were dried over Na₂SO₄ and filtrated. The filtrate evaporated in the rotary vacuum evaporator and the residuum was separated by chromatography (silica gel, ethylacetate-hexane=1:1). **1a** and **2r** in the form of a light yellow oil (920 mg, 88 %, d.r.: *syn: anti* 81:19, e.e.: *syn*: 97 %, *anti:* 56 %) were obtained together.

### Example 7

### Preparation of N-[(2R,3S)-1-nitro-4-oxo-3-(pentane-3-yloxy)butane-2-yl]acetamide (1)

2.317 g (17.80 mmol) of pent-3-yloxyacet-aldehyde (1.1 equiv.) was added to the suspension of 2.105 g (16.18 mmol) of N-(Z)-2-nitroethenylacetamide in 40 ml of chloroform at the temperature of 0 °C and then the solution of 0.527 g (1.618 mmol, 0.1 equiv.) of (2S)-2-(diphenyl[(trimethylsilyl)oxy]methyl)pyrrolidine in 25 ml of chloroform and the solution of 0.449 g (3.236 mmol, 0.2 equiv.) of bromoacetic acid in 65 ml of water was added. The reaction mixture was stirred at the temperature of 0 °C. After 1 hour 0.421 g (3.236 mmol) of pent-3-yl-oxyacetaldehyde (0.2 equiv.) was added and after another hour 0.421 g (3.236 mmol) of pent-3-yloxy-acetaldehyde (90.2 equiv.) was added. The reaction mixture was stirred for 3.5 hours in total at the temperature of 0 °C. After termination of the reaction 80 ml of saturated cooled solution of NH₄Cl was added to the reaction mixture. After thorough stirring the chloroform layer was separated in separatory funnel, the water layer was extracted by chloroform (3x50 ml) and joined organic layers were dried by anhydrous Na₂SO₄. After drying the solution was evaporated and the residuum was separated by chromatography (silica gel; ethylacetate:i-hexane = 1:1), thus obtaining 1.956 g (46.5 %) of **(1)** with 97% ee as, light yellow oil and 461 mg (11%) of anti diastereoisomer (69 %ee).

### Example 8

### Preparation of N-[(2R,3S)-1-nitro-4-oxo-3-(pentane-3-yloxy)butane-2-yl]acetamide (1 b) and N-[(2S,3S a 2R, 3R)-1-nitro-4-oxo-3-(pentane-3-yloxy)butane-2-yl]acetamide (2r) using various catalysts

The reactions were performed in deuterated chloroform and conversion of the starting nitro-olefine and ratio between diastereomers from NMR measurements (300MHz) was determined in the reaction mixture, as well as enentiomeric excess of diastereoisomers after derivatization of the reaction mixture according to the procedure described in Example 8.

### General procedure:

The co-catalyst (6, 6a-6k, see below) (0.2 equiv.) was dissolved in water and the catalyst (2S)-2-(diphenyl[(trimethylsilyl)oxy]methyl)pyrrolidine dissolved in CDCl3 was added to the suspension of 2-nitroethenylacetamide (0.25 mmol) and pent-3-yloxyacetaldehyde (1.5 equiv.) in 0.75 ml of CDCl3, and the mixture was stirred at the temperature of 0 °C for 2 hours. After termination, a sample was taken directly from the reaction mixture for NMR determination of conversion and dr. The obtained crude products were further derivatized in order to determine the enantiomeric excess. The results are summarized in the table below:

| **Input** | **Co-catalyst** | **Conversion in%** | **dr syn/anti** | **ee syn %** | **ee anti %** | **Note** |
|---|---|---|---|---|---|---|
| 1 | 6b | 97 | 1.7:1 | - | - | 0.2 equiv. of co-catalyst, cooling to -10 °C |
| 2 | 6c | 90 | 2.3:1 | - | - | 0.2 equiv. of co-catalyst |
| 3 | 6c | 92 | 2.5:1 | 90 | 86 | 0.3 equiv. of co-catalyst, 2 equiv. of aldehyde, 22 hours |
| 4 | 6 | 97 | 4.3:1 | 95.2 | 76.6 | |
| 5 | 6a | 97 | 3.8:1 | 96.6 | 68.2 | |
| 6 | 6d | 94 | 3.6:1 | 98.4 | 76.6 | |
| 7 | 6e | 98 | 4:1 | 97 | 77 | |
| 8 | 6f | 60 | 4.7:1 | 89.7 | 78.7 | |
| 9 | 6g | 90 | 3:1 | - | - | |
| 10 | 6h | 69 | 3.2:1 | 90.4 | 65.2 | |
| 11 | 6i | 95 | 4.1:1 | 95 | 57.6 | Added 0.5 equiv. excess of aldehyde |
| 12 | 6 | 99.6 | 4.6:1 | 97 | 66 | Added 0.5 equiv. excess of aldehyde |
| 13 | 6a | 94 | 4.6:1 | 96.2 | 69.6 | Added 0.5 equiv. excess of aldehyde |
| 14 | 6d | 99.8 | 3.2:1 | 96.8 | 69.6 | Added 0.5 equiv. excess of aldehyde |
| 15 | 6k | 48 | 5.9:1 | 90.1 | 84.6 | Reaction time 1 hour |
| 16 | 6e | 82.3 | 4.7:1 | 90.4 | 93.4 | 0.25 equiv. of co-catalyst, reaction time 1 hour |
| 17 | 6 | 99 | 3.7:1 | 95.8 | 66.1 | 0.3 equiv. of co-catalyst, reaction time 1 hour |
| 18 | 6 | 92.5 | 4.5:1 | 96.5 | 80.8 | Reaction time 7 hours¹ |
| 19 | 6 | 43 | 4.1:1 | 97 | 89 | Reaction time 22 hours² |
| 20 | 6a | 95 | 5.1:1 | 96.5 | 60.1 | Reaction time 4 hours³ |
| 21 | 6a | 96.2 | 5.2:1 | 96.2 | 58.2 | Reaction time 4 hours⁴ |
| 22 | 6a | 92.7 | 5.4:1 | 96.9 | 59.9 | Reaction time 4 hours⁵ |
| 23 | 6j | 72 | 3:1 | 90 | 69 | Solvent CDCl₃: H₂O, reaction time 24hours |
| 24 | 6c | 65 | 2.9:1 | 89.5 | 69.7 | Solvent CDCl₃, -5 °C, 3 hours⁶ |
| 25 | 6 | 61 | 5.9:1 | 95.9 | 75.6 | Solvent CDCl₃, -5 °C, 3 hours⁶ |

| | | | | | | |
|---|---|---|---|---|---|---|
| ¹aldehyde 2.75 equiv., catalyst 0.2 equiv., co-catalyst 0.505 equiv., H₂O : CDCl₃ = 1 : 1.5 ml, 0°C ²aldehyde 0.3 equiv., co-catalyst 0.3 equiv., anhydrous ³aldehyde 1.86 equiv., co-catalyst 0.1 equiv., H₂O 0.82 ml, CDCl₃ 0.51 ml ⁴aldehyde 1.92 equiv., co-catalyst 0.086 equiv., H₂O 0.87 ml, CDCl₃ 0,47 ml ⁵aldehyde 1.98 equiv., co-catalyst 0.071 equiv., catalyst 0.07 equiv., H₂O 0.91 ml, CDCl₃ 0.44 ml ⁶aldehyde 2 equiv., co-catalyst 0.3 equiv. 6 - monochloracetic acid 6a - bromoacetic acid 6b - acetic acid 6c - benzoic acid 6d - iodoacetic acid 6e - mandelic acid 6f - 1-bromophenylacetic acid 6g - phenylacetic acid 6h - 4-bromophenylacetic acid 6i - fluoroacetic acid 6j - 2-chloropropionic acid 6k - 4-chloromandelic acid | | | | | | |

### Example 9

### Preparation of N-[(4-methoxyphenyl)methyl]-N-[(2R,3S and 2S,3R)-1-nitro-4-oxo-3-(pentane-3-yloxy)butane-2-yl]acetamide (7r) and N-[(4-methoxyphenyl)methyl]-N-[(2R,3R and 2S.3S)-1-nitro-4-oxo-3-(pentane-3-yloxy)-butane-2-yl]acetamide (8r)

500 mg (2 mmol) of N-[(4-methoxyphenyl)methyl]-N-[(E)-2-nitroethenyl]acetamide and then 390 mg (3 mmol) of 2-(pentane-3-yloxy)acet-aldehyde was added to the solution of 260 mg (0.8 mmol) of (2*R*)-2-(diphenyl[(trimethylsilyl)-oxy]methyl)pyrrolidine in chloroform (8ml) at the temperature of 0 °C. The reaction mixture was stirred for 90 hours at room temperature, then it was directly evaporated in the rotary vacuum evaporator and chromatographed (silica gel, hexane:ethylacetate = 2:1). 400 mg (53 %) of light oil as a mixture of two diastereoisomers **7r, 8r** (dr = 1:1.1. ee% = 53 % and 41 %) was obtained.

**(7r, 8r):** 1 H NMR (300 MHz, CDCl₃, in two isomers, 56H): δ 0,72-0,88 (m, 12H), 1,26-1,55 (m, 8H), 2,20 (s, 3H), 2,21 (s, 3H), 3,01-3,09 (m, 1 H), 3,15-3,23 (m, 1 H), 3,81 (s, 6H), 3,90 to 3,93 (m, 1 H), 4,25 to 4,64 (m, 5H), 4,76-4,84 (m, 5H), 4,94-5,01 (m, 1 H), 6,89-6,93 (m, 4H), 7,12-7,19 (m, 4H), 9,15 (d, J = 3,3 Hz, 1 H), 9,51 (d , 3,0 Hz, 1 H), *MS: m* /*z* [M+H⁺] calculated for: [C19H28N2O6] 381,20; found 381,19

### Example 10

### Preparation of N-[(4-methoxyphenyl)methyl]-N-[(2R,3S a 2S,3R)-1-nitro-4-oxo-3-(pentane-3-yloxy)butane-2-yl]acetamide (7r) and N-[(4-methoxyphenyl)methyl]-N-[(2R,3R a 2S.3S)-1-nitro-4-oxo-3-(pentane-3-yloxy)butane-2-yl]acetamide (8r)

Chloroform (4 ml), N-[(4-methoxyphenyl)methyl]-N-[(E)-2-nitroethenyl]acetamide (250 mg, 1 mmol), 2-(pentane-3-yloxy)acetaldehyde (195 mg, 1.5 mmol) and monochloroacetic acid (38 mg, 0.4mmol) were added to (*2R*)-2-(diphenyl[(trimethylsilyl)oxy]methyl)pyrrolidine (130 mg, 0.4 mmol). The reaction mixture was stirred for 120 hours at room temperature. Then it was directly evaporated in the rotary vacuum evaporator and chromatographed on silica gel (hexane:ethylacetate = 2:1). 95 mg (25 %) of light oil as a mixture of two diastereoisomers **7r, 8r** (dr = 1:1.2. ee = 80% a 87 %) was obtained.

### Example 11

### Preparation of N-[(2S,3R and 2R,3S)-3-(benzyloxy)-1-nitro-4-oxobutane-2-yl]acetamide (9r) and N-[(2R,3R and 2S,3S)-3-(benzyloxy)-1-nitro-4-oxobutane-2-yl]acetamide (10r)

Benzyloxyacetaldehyde (121.2 mg, 0.807 mmol) was added to the solution of N-[(Z)-2-nitro-ethenyl]acetamide (70 mg, 0.538 mmol) and (2S-2-(diphenyl[(trimethylsilyl)oxy]methyl)pyrrolidine (17.5 mg, 0.0538 mmol) in dimethyl sulfoxide (2 ml) at room temperature. Then monochloroacetic acid (10.3 mg, 0.107 mmol) was added, and the reaction mixture was stirred for 24 hours at room temperature. The solution was diluted by water (10 ml) and extracted by EtOAc (3x10 ml). The joined organic layers were washed by saturated solution of NaCl and dried by anhydrous Na₂SO₄. Following the chromatography (silica gel, EtOAc:n-hexane=1:3 to 1:1) the final product as a light orange oil corresponding to the mixture of diastereoisomers **9r** and **10r** (112 mg, 74 %) was obtained.

**(9r, 10r)**: 1 HNMR: (300 MHz, CDCl₃, TMS): δ = 9,60 (s, 1 H), 9,56 (d, J = 2,5, 1 H), 7,36 (m, 5H), 6,02 (m, 1 H), 4,80 (d, J = 11,3, 1 H), 4,71 (dd, J = 6,7, J = 8,6, 1 H), 4,56 (m, 2H), 4,50 (m, 1 H), 4,15 (m, 1 H), 1,94 (d, J = 8,9, 3H). *MS: m*/*z* [M+H⁺] calculated for [C₁₃H₁₆N₂O₅⁺]: 281,11; found: 281,10.

### Example 12

### Preparation of N-[(2S,3R and 2R,3S)-1-nitro-4-oxo-3-(pentane-3-yloxy)butane-2-yl]acetamide (1r) and N-[(2S,3S and 2R, 3R)-1-nitro-4-oxo-3-(pentane-3-yloxy)butane-2-yl]acetamide (2r) using (2S)-2-(methoxymethyl)pyrrolidine as a catalyst

Monochloroacetic acid (0.4 equiv., 19 mg) and (2S)-2-(methoxymethyl)pyrrolidine (0.1 equiv., 6 mg) in CHCl₃ (1 ml) were added to the suspension of N-[(Z)-2-nitroethenyl]acetamide (0.5 mmol, 65 mg), and then 1-ethylpropyloxyacetaldehyde (1.5 equiv., 130 mg) in the mixture of CHCl₃ (2 ml) and water (2 ml) was added at room temperature. The reaction mixture was stirred at room temperature for 48 hours. After the reaction terminated, the saturated solution of NH₄Cl (3 ml) was added and the chloroform layer was separated. The water layers was extracted by chloroform (5x2ml). The joined organic layers were dried by anhydrous Na₂SO₄ and after filtration the filtrate was evaporated in the rotary vacuum evaporator. The crude product was separated by chromatography (silica gel, ethylacetate:hexane=1:1). A ligh brown oil as a mixture of diastereoisomers **1r** and **2r** (40 mg, 30 %, dr: *syn:anti* 81:19, ee: *syn:* 46 %, *anti:* 65 %) was obtained.

### Example 13

### Preparation of N-[(2S,3R and 2R,3S)-1-nitro-4-oxo-3-(pentane-3-yloxy)butane-2-yl]acetamide (1 r) and N-[(2S,3S and 2R, 3R)-1-nitro-4-oxo-3-(pentane-3-yloxy)butane-2-yl]acetamide (2r) using (2S)-2-(methoxydiphenylmethyl)pyrrolidine as a catalyst

Monochloroacetic acid (0.4 equiv., 19 mg) and (2S)-2-(methoxydiphenylmethyl)-pyrrolidine (0.1 equiv., 13 mg) in CHCl₃ (1 ml) were added to the suspension of N-[(Z)-2-nitroethenyl]acetamide (0.5 mmol, 65 mg) and then 1-ethylpropyloxyacetaldehyde (1.5 equiv., 130 mg) in the mixture of CHCl₃ and water (1:1) (4 ml) was added at room temperature. The reaction mixture was stirred at room temperature for 1.5 hours. After the reaction terminated, the saturated solution of NH₄Cl (3 ml) was added and chloroform layer was separated. The water layer was extracted by chloroformom (5x1 ml). The joined organic layers were dried by anhydrous Na₂SO₄ and after filtration the filtrate was evaporated in the rotary vacuum evaporator. The crude product was separated by chromatography (silica gel, ethylacetate:hexane=1:1). A light brown oil as a mixture of diastereoizomers **1r** and **2r**, 100 mg, 76 %, dr: *syn:anti* 70:30, ee: *syn:* 86 %, *anti:* 77 %) was obtained.

### Preparation of 2-nitroethenylamides

### Example 14

### Preparation of N-[(Z)-2-nitroethenyl]acetamide (11)

Acetanhydride (2 equiv., 3.7 ml) was added to the solution of 2-nitroethenylamine (1.76 g, 20 mmol) in pyridine (15 equiv., 24 ml) at the temperature of 0-5 °C during 5 min. Then the reaction mixture was stirred at room temperature for 28 hours. The reaction mixture was added to water solution of CuSO₄ (200 ml) and the obtained mixture was extracted by ether (5x 80 ml). The organic layers were dried with Na₂SO₄ and ether evaporated in the rotary vacuum evaporator. Following the chromatography (silicagel,ethylacetate:hexane=1:1) N-[(Z)-2-nitro-ethenyl]acetamide **(11)** (2216 mg, 85.24 %) in the form of white crystals was obtained. MP (melting point) = 117-119 °C
**(11):** ¹H NMR, (300 MHz, CDCl₃) δ 10,6 to 10,2 (bs, 1 H), 7,59 (dd, J = 7,0, 12,3 Hz, 1 H), 6,61 (d, J = 6,7 Hz, 1 H), 2,28 (s, 3H) *MS*: *mlz* [M+H⁺] calculated for: [C₄H₇N₂O₃] = 131,11; found: 131,04

### Example 15

### Preparation of 2-methyl-N-[(E)-2-nitroethenyl]propanamide (12)

2-methylpropanoyl-2-methylpropanoate (4 equiv., 11.3 ml) was added to the solution of 2-nitro-ethenylamine (1.5 g, 17 mmol) in pyridine (15 equiv., 20 ml) at the temperature of 0-5 °C during 5 min. Then the reaction mixture was stirred at room temperature for 48 h. The reaction mixture was added to water solution of CuSO₄ (200 ml) and the obtained mixture was extracted by ether (5x 80 ml). The organic layers were dried with Na2SO4 and ether evaporated in the rotary vacuum evaporator. Following the chromatography (silica gel, ethylacetate:hexane=1:1) 2-methyl-N-[(E)-2-nitroethenyl]propanamide **(12)** (2330mg, 86 %) in the form of light crystals with MP 92-94 °C was obtained.
**(12):** 1H NMR, (300 MHz, CDCl₃) δ 8,40 (dd, J = 11,1, 12,1 Hz, 1H), 7,72 to 7,55 (bs, 1 H), 7,42 (d, J = 12,1 Hz, 1 H), 2,56 (trans J = 6,9 Hz, 1 H), 1,25 (d, J = 6,9 Hz, 1 H), *MS*: m/z [M+H⁻] calculated for: [C₆H₁₁N₂O₃] = 157,06; found: 157,05

### Example 16

### Preparation of 2,2-dimethyl-N-[(Z)-2-nitroethenyl]propanamide (13)

Triethylamine (3 equiv., 3.7 ml) and pivaloyl chloride (1.1 equiv., 1.64 ml) were added to the solution of 2-nitroethenylamine (1000 mg, 12 mmol) in dichloromethane (60 ml) at the temperature of 0-5 °C for 2 min. Then the reaction mixture was stirred at room temperature 23 hours. After termination of the reaction, silica gel (6 g) was added to the reaction mixture and the solvent evaporated in the rotary vacuum evaporator. The obtained solid residuum was chromatographed (silica gel, ethylacetate:hexane=1:4), thus obtaining 2,2-dimethyl-N-[(Z,E)-2-nitroethenyl]propanamide **(13)** (1510 mg, 73 %) as a light yellow oil.
**(13):** ¹H NMR, (300 MHz, CDCl₃) (Z: E = 2:1), δ 10,95 to 10,75 (bs, 1 H, (Z)), 8,41 (dd, J = 10,9, 12,1 Hz, 1 H, (E)), 7,82 to 7,72 (bs, 1 H, (E)), 7,63 (dd, J = 6,8 a 11,9 Hz, 1 H, (Z)), 7,45 (d, J = 12,1 Hz, 1 H, (E)), 6,64 (d, J = 6,8 Hz, 1 H, (Z)), 1,32 (s, 9H, (Z)), 1,29 (s , 9H, (E)), *MS*: *m*/*z* [M-H⁻] calculated for: [C₇H₁₃N₂O₃] = 171,07; found: 171,07

### Example 17

### Preparation of N-[(E)-2-nitroethenyl]-N-(prop-2-en-1-yl)acetamide (14)

Acetanhydride (4 equiv., 3.3 ml) was added to the solution of N-[(Z)-2-nitroethenyl]-N-(prop-2-en-1-yl)amine (1.5 g, 7.5 mmol) in pyridine (15 equiv., 14 ml) at the temperature of 0-5 °C during 5 min. Then the reaction mixture was stirred at room temperature for 48 hours. The reaction mixture was added to water solution of CuSO₄ (150 ml) and the obtained mixture was extracted by ether (5x 40 ml). The joined organic layers were dried by Na₂SO₄, ether evaporated in the rotary vacuum evaporator. Following the chromatography (silica gel, ethylacetate:hexane=1:3), N-[(Z)-2-nitroethenyl]-N-(prop-2-en-1-yl)acetamide **(14)** (1155 mg, 91 %) in the form of a light yellow oil was obtained.
**(14):** ¹H NMR, (300 MHz, CDCl₃) δ 8,75 to 8,55 (bd, 1 H), 6,93 (d, J = 11,8 Hz, 1 H), 5,79 to 5,67 (m, 1 H), 5,31 (td, J = 1,4, 10,5 Hz, 1 H), 5,21 to 5,15 (m, 1 H), 4,27 to 4,24 (m, 2H ), 2,42 (s, 3H)

### Example 18

### :N-[(4-metoxyphenyl)methyl]-N-[(E)-2-nitroethenyl]acetamide (15)

2350 mg (23 mmol) of acetanhydride was added to [(4-metoxyphenyl)methyl][(E)-2-nitroethenyl]amine (19.2 mmol, 4000 mg) dissolved in 70 ml of pyridine at the temperature of 0 °C during 15 minutes. The reaction mixture was stirred at room temperature for 16 hours. Then this mixture was poured into water (500 ml) and the mixture was extracted by 3 x 100 ml ethylacetate. The joined organic layers were dried over Na₂SO₄, the solvent evaporated in the rotary vacuum evaporator and the residuum (4.25 g) was purified by chromatography (silica gel, ethylacetate:hexane=2:3). 4000 mg N-[(4-metoxyphenyl)methyl]-N-[(E)-2-nitroethenyl] acetamide **(15)** as a yellow solid was obtained.
**(15)** :¹H NMR (300 MHz, CDCl₃): δ 2,48 (s, 3H), 3,80 (s, 3H), 4,79 (s, 2H), 6,87-6,91 (m, 3 h), 7,06-7,11 (m, 2H), 8,66 (bs, 1 H). Calculated for C₁₂H₁₄N₂O₄: 57,59 % C, 5,64 % H, 11,19 % N; found: 57,83 % C, 5,74 % N,11,43 % N

### Example 19

### Preparation of (Z)-N-benzyl-N-(2-nitrovinyl)acetamide (16)

Dimethylaminopyridine (6 mmol), triethylamine (30 mmol) and acetanhydride (36 mmol) were added to the suspension of 2-nitroethenylbenzylamine (30mmol) in CH₂Cl₂ (100 ml) at the temperature of 0 °C. Then the reaction mixture was left to be spontaneously heated to the room temperature and stirred for 3 hours. Water was added to the reaction mixture and the organic layer was separated. The water layer was extracted by CH₂Cl₂. The joined organic layers were washed by saturated solution of NaCl and dried over Na₂SO₄. After evaporation of the solvent in the vacuum, the residuum was purified by chromatography on silica gel, thus obtaining (Z)-N-benzyl-N-(2-nitrovinyl)acetamide **(16)** (74 %) as a yellow solid.
**(16):** ¹H NMR (400 MHz, CDCl₃) δ: 8,68 (br, 1 H), 7,18 to 7,38 (m, 5 H), 6,84 (d, *J* = 11,6 Hz, 1 H), 4,86 (s, 2 H), 2,49 (s, 3H).
*Note:*
*The work leading to this invention was made under the Seventh Framework Programme of the European Union, the number of the Grant Agreement: 201431*

## Claims

1. Process for the preparation of 1-nitro-4-oxobutanylamides of general formula la and Ib 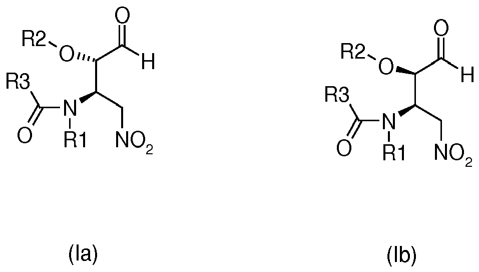 or opposite enantiomers thereof,
wherein R1 is H, allyl, alkyloxycarbonyl, arylalkyloxycarbonyl with a number of carbon atoms up to 24 or the same as in R2, R2 represents alkyl, cycloalkyl or phenylalkyl with up to 24 carbon atoms, and R3 represents alkyl, alkylphenyl, phenylalkyl or phenyl, substituted phenyl, alkoxyphenylalkyl with up to 24 carbon atoms, or wherein R3 is methyl when R1 is 4-methoxyphenylmethyl, **characterized in that** aldehydes of general formula II, 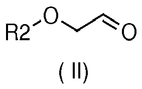 wherein R2 is defined as above,
reacts with 2-nitroethenylamides of general formula IIIa and/or IIIb, 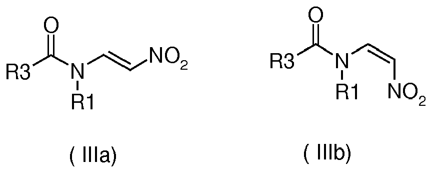 wherein R1 and R3 are defined as above,
in the presence of organic catalysts and co-catalysts in organic solvent, water, in solution of inorganic salts in water or in the mixture of organic solvents and water or in the mixture of solutions of inorganic salts in water and organic solvents at the temperature between -20 and +50 °C.

2. Process according to claim 1, **characterized in that** the organic catalyst is a pyrrolidine derivative of general formula IV and V, 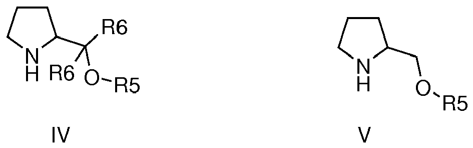 wherein R6 is phenyl or substituted phenyl with up to 12 carbon atoms, and R5 is alkyl with 1 to 4 carbon atoms or trimethylsilyl.

3. Process according to claim 1 or 2, **characterized in that** the organic catalyst is (2*S*)-2-(diphenyl[(trimethylsilyl)oxy]methyl)pyrrolidine or (2*R*)-2-(diphenyl[(tri-methylsilyl)oxy]methyl)pyrrolidine.

4. Process according to any of claims 1 to 3, **characterized in that** the co-catalysts are carboxylic acids with 1 to 12 carbon atoms or carboxylic acids substituted with halogen, aromatic acids, phenylalkane carboxylic acids, cycloalkanecarboxylic acids with 6 to 12 carbon atoms or several other organic acids with 1 to 12 carbon atoms.

5. Process according to claim 4, **characterized in that** the co-catalyst is monochloroacetic acid, bromoacetic acid or iodoacetic acid.

6. Process according to claim 1, **characterized in that** the solvents are organic solvents, water, water with inorganic salts or mixtures of organic solvents with water or with solution of inorganic salts in water.

7. Process according to claim 6, **characterized in that** the solvents are alcohols with 1 to 5 carbon atoms, water, mixture of water with dichloromethane or chloroform or solution of inorganic salts in water with dichloromethane or chloroform.

8. Process according to claim 1, **characterized in that** the temperature is between -15 and 25 °C.

9. 2-nitroethenylamides of general formula IIIa and IIIb 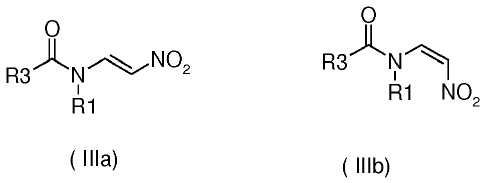 wherein R1 is H, allyl, alkyloxycarbonyl, arylalkyloxycarbonyl with a number of carbon atoms up to 24 or alkyl, cycloalkyl or phenylalkyl with up to 24 carbon atoms, and R3 represents alkyl, alkylphenyl, phenylalkyl or phenyl, alkoxyphenylalkyl with up to 24 carbon atoms or wherein R3 is methyl when R1 is 4-methoxyphenylmethyl.

10. Compounds according to claim 9, **characterized in that** R1 is H and R3 is methyl.

11. Process for the preparation of 2-nitroethenylamides of general formula IIIa and IIIb according to claim 9, 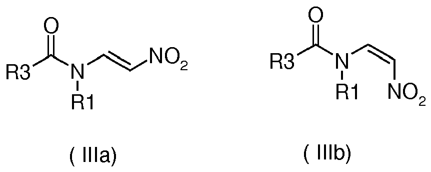 or mixtures thereof,
wherein R1 is H, allyl, alkyloxycarbonyl, arylalkyloxycarbonyl with a number of carbon atoms up to 24, R3 is alkyl, cycloalkyl, phenyl or alkylphenyl, alkoxyphenylalkyl with up to 24 carbon atoms, or wherein R3 is methyl when R1 is 4-methoxyphenylmethyl,
**characterized in that** 2-nitroethenylamine of general formula VIa and/or VIb, 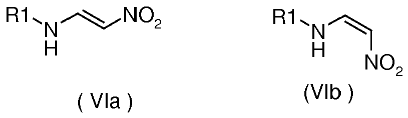 wherein R1 is defined as above,
reacts with corresponding carboxylic acids or derivatives thereof with 1 to 24 carbon atoms.

## Patentansprüche

1. Verfahren zur Bereitstellung von 1-Nitro-4-oxobutanylamiden der allgemeinen Formel Ia und Ib 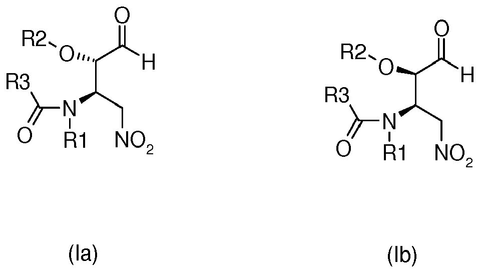 oder deren umgekehrten Enantiomeren,
worin R1 für Wasserstoff, Allyl, Alkyloxycarbonyl, Arylalkyloxycarbonyl mit bis zu 24 Kohlenstoffatomen steht oder gleich wie R2 ist, R2 für Akyl, Cycloalkyl oder Phenylalkyl mit bis zu 24 Kohlenstoffatomen steht und R3 für Alkyl, Alkylphenyl, Phenylalkyl oder Phenyl, substituiertes Phenyl, Alkoxyphenylalkyl mit bis zu 24 Kohlenstoffatomen steht,
**dadurch gekennzeichnet, dass** man die Aldehyde der allgemeinen Formel II, 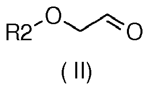 worin R2 wie oben definiert ist,
mit 2-Nitroethenylamiden der allgemeinen Formel IIIa und/oder IIIb, 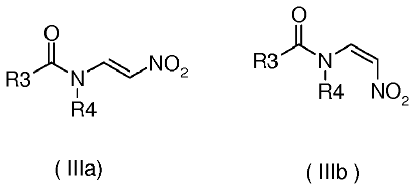 worin R1 und R3 wie oben definiert sind, in der Gegenwart von organischen Katalysatoren und Co-katalysatoren in einem organischen Lösungsmittel, im Wasser, in einer wässrigen Lösung der anorganischen Salze oder im Gemisch von organischen Lösungsmittel mit Wasser oder im Gemisch der wässrigen Lösungen der anorganischen Salze und organischer Lösungsmittel bei einer Temperatur von -20 bis +50 °C umsetzt.

2. Verfahren nach Anspruch 1, **dadurch gekennzeichnet, dass** der organische Katalysator ein Pyrrolidinderivat der allgemeinen Formel IV und V ist, 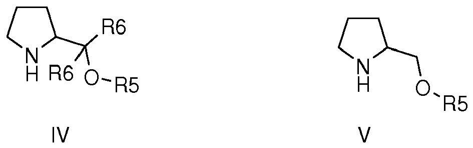 worin R6 für Phenyl oder substituiertes Phenyl mit bis zu 12 Kohlenstoffatomen steht, R5 für Alkyl mit 1 bis 4 Kohlenstoffatomen oder Trimethylsilyl steht.

3. Verfahren nach Anspruch 1 oder 2, **dadurch gekennzeichnet, dass** der organische Katalysator ein (2*S*)-2-(Diphenyl[(trimethylsilyl)oxylmethyl)pyrrolidin oder (2R)-2-(Diphenyl[(trimethylsilyl)oxy]methyl)pyrrolidin ist.

4. Verfahren nach einem der Ansprüche 1 bis 3, **dadurch gekennzeichnet, dass** die Co-katalysatoren die Carbonsäuren mit 1 bis 12 Kohlenstoffatomen oder halogensubstituierte Carbonsäuren, aromatische Säuren, Phenylalkancarbonsäuren, Cycloalkancarbonsäuren mit 6 bis 12 Kohlenstoffatomen oder einige weitere organische Säuren mit 1 bis 12 Kohlenstoffatomen sind.

5. Verfahren nach Anspruch 4, **dadurch gekennzeichnet, dass** Co-katalysator die Chloressigsäure, Bromessigsäure oder Iodessigsäure ist.

6. Verfahren nach Anspruch 1, **dadurch gekennzeichnet, dass** die Lösungsmittel die organische Lösungsmittel, Wasser, Wasser mit anorganischen Salzen oder Gemische der organischen Lösungsmittel mit Wasser oder mit einer Lösung der anorganischen Salze im Wasser sind.

7. Verfahren nach Anspruch 6, **dadurch gekennzeichnet, dass** die Lösungsmittel die Alkohole mit 1 bis 5 Kohlenstoffatomen, Wasser, Gemisch aus Wasser mit Dichlormethan oder Chloroform oder eine Lösung der anorganischen Salze im Wasser mit Dichlormethan oder Chloroform sind.

8. Verfahren nach Anspruch 1, **dadurch gekennzeichnet, dass** die Temperatur im Bereich von -15 bis 25 °C liegt.

9. 2-Nitroethenylamide der allgemeinen Formel IIIa und IIIb 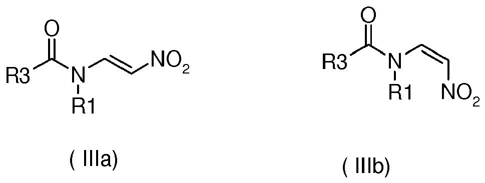 worin R1 für H, Allyl, Alkyloxycarbonyl, Arylalkyloxycarbonyl mit bis zu 24 Kohlenstoffatomen oder Alkyl, Cycloalkyl oder Phenylalkyl mit bis zu 24 Kohlenstoffatomen steht und R3 Alkyl, Alkylphenyl, Phenylalkyl oder Phenyl, Alkoxyphenylalkyl mit bis zu 24 Kohlenstoffatomen darstellt und wobei R3 für Methyl steht, falls R1 ein 4-Methoxyphenylmethyl ist.

10. Verbindungen nach Anspruch 9, **dadurch gekennzeichnet, dass** R1 für H und R3 für Methyl steht.

11. Verfahren zur Bereitstellung von 2-Nitroethenylamiden der allgemeinen Formel IIIa und IIIb nach Anspruch 9, 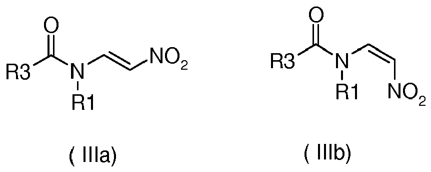 oder deren Gemische,
worin R1 für H, Allyl, Alkyloxycarbonyl, Arylalkyloxycarbonyl mit bis zu 24 Kohlenstoffatomen steht, R3 für Alkyl, Cycloalkyl, Phenyl oder Alkylphenyl, Alkoxyphenylalkyl mit bis zu 24 Kohlenstoffatomen steht, oder wobei R3 ein Methyl bedeutet, falls R1 ein 4-Methoxyphenylmethyl ist,
**dadurch gekennzeichnet, dass** man 2-Nitroethenylamin der allgemeinen Formel VIa und/oder VIb 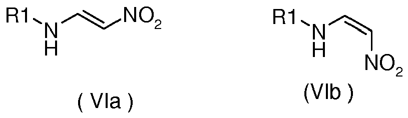 worin R1 wie oben definiert ist,
mit entsprechenden Carbonsäuren oder deren Derivaten mit 1 bis 24 Kohlenstoffatomen umsetzt.

## Revendications

1. Procédé de préparation des 1-nitro-4-oxobutanylamides de formule générale Ia et Ib 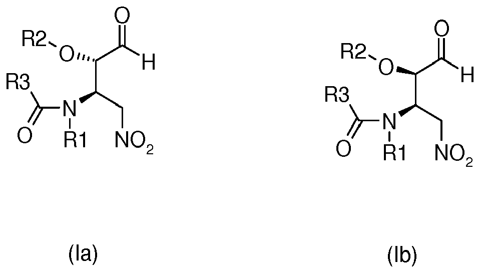 ou des énantiomères opposés de celles-ci,
dans lesquelles R1 représente H, allyle, alkyloxycarbonyle, arylalkyloxycarbonyle ayant le nombre d'atomes de carbone jusqu'à 24 ou est identique à R2, R2 représente alkyle, cycloalkyle ou phénylalkyle ayant le nombre d'atomes de carbone jusqu'à 24 et R3 représente alkyle, alkylphényle, phénylalkyle ou phényle, phényle substitué, alcoxyphénylalkyle ayant le nombre d'atomes de carbone jusqu'à 24, ou dans lesquelles R3 est méthyle et R1 est 4-méthoxyphénylméthyle.
characterisé en ce que les aldéhydes de formule générale II, 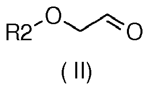 dans laquelle R2 est tel que défini ci-dessus,
réagissent avec les 2-nitroéthenylamides de formule générale IIIa et/ou IIIb, 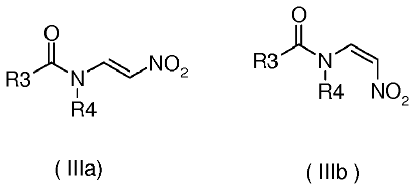 dans lesquelles R1 et R3 sont tels que définis ci-dessus,
en présence des catalyseurs et cocatalyseurs organiques dans un solvant organique, l'eau, une solution de sels inorganiques dans l'eau ou dans un mélange de solvants organiques et d'eau ou dans un mélange de solutions de sels inorganiques dans l'eau et des solvants organiques à la température de -20 à +50 °C.

2. Procédé selon la revendication 1, characterisé en ce que le catalyseur organique est un dérivé de pyrrolidine de formule générale IV et V, 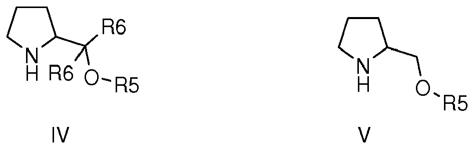 dans lesquelles R6 représente phényle ou phényle substitué ayant le nombre d'atomes de carbone jusqu'à 12, R5 représente alkyle ayant 1 à 4 atomes de carbone ou triméthylsilyle.

3. Procédé selon la revendication 1 ou 2, characterisé en ce que le catalyseur organique est (2*S*)-2-(diphényl[(triméthylsilyl)oxy]méthyl)pyrrolidine ou (2R)-2-(diphényl[(triméthylsilyl)oxy]méthyl)pyrrolidine.

4. Procédé selon l'une quelconque des revendications 1 à 3, characterisé en ce que les cocatalyseurs sont les acides carboxyliques ayant 1 à 12 atomes de carbone ou les acides carboxyliques substitués par un halogène, les acides aromatiques, les acides phénylalcanecarboxyliques, les acides cycloalcanecarboxyliques ayant 6 à 12 atomes de carbone ou des plusieurs autres acides organiques ayant 1 à 12 atomes de carbone.

5. Procédé selon la revendication 4, characterisé en ce que le cocatalyseur est l'acide chloroacétique, bromoacétique ou iodoacétique.

6. Procédé selon la revendication 1, characterisé en ce que les solvants sont les solvants organiques, l'eau, l'eau comprenant des sels inorganiques ou les mélanges de solvants organiques et d'eau ou de solution de sels inorganiques dans l'eau.

7. Procédé selon la revendication 6, characterisé en ce que les solvants sont les alcools ayant 1 à 5 atomes de carbone, l'eau, un mélange d'eau et de dichlorométhane ou chloroforme ou de solution de sels inorganiques dans l'eau avec dichlorométhane ou chloroforme.

8. Procédé selon la revendication 1, characterisé en ce que la température est entre -15 à 25 °C.

9. 2-nitroéthenylamides de formule générale IIIa et IIIb 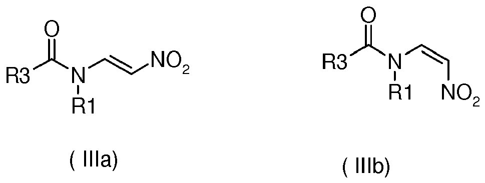 dans lesquelles R1 représente H, allyle, alkyloxycarbonyle, arylalkyloxycarbonyle ayant le nombre d'atomes de carbone jusqu'à 24 ou alkyle, cycloalkyle ou phénylalkyle ayant le nombre d'atomes de carbone jusqu'à 24 et R3 représente alkyle, alkylphényle, phénylalkyle ou phényle, alcoxyphénylalkyle ayant le nombre d'atomes de carbone jusqu'à 24 et dans lequels R3 est méthyle quand R1 est 4-méthoxyphénylméthyle.

10. Composés selon la revendication 9, characterisés en ce que R1 est H et R3 est méthyle.

11. Procédé de préparation des 2-nitroéthenylamides de formule générale IIIa et IIIb selon la revendication 9, 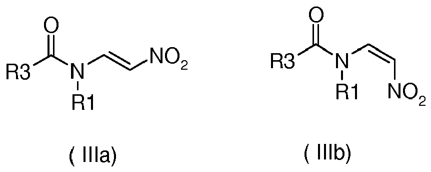 ou des mélanges de ceux-ci dans lesquelles R1 représente H, allyle, alkyloxycarbonyle, arylalkyloxycarbonyle ayant le nombre d'atomes de carbone jusqu'à 24, R3 représente alkyle, cycloalkyle, phényl ou alkylphényle, alcoxyphénylalkyle ayant le nombre d'atomes de carbone jusqu'à 24, ou dans lesquelles R3 est méthyle quand R1 est 4-méthoxyphénylméthyle,
characterisé en ce que 2-nitroéthenylamine de formule générale VIa et/ou VIb 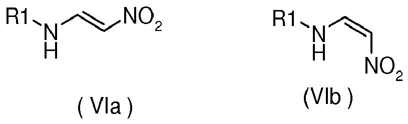 dans lesquelles R1 est tel que défini ci-dessus,
réagit avec les acides carboxyliques correspondants ou les dérivés de ceux-ci ayant 1 à 24 atomes de carbone.
